# FASCICULE DE BREVET EUROPEEN

(11) **EP 2 523 940 B1**
(45) Date de publication et mention de la délivrance du brevet: **12.10.2016**
(21) Numéro de dépôt: 11704265.5
(22) Date de dépôt: 07.01.2011
(51) Int. Cl.: C07D 233/36, C07D 233/42

(54) **PROCÉDÉ DE PRÉPARATION D'AMINOÉTHYL IMIDAZOLIDINONE OU DE SON THIOCARBONYLE**
VERFAHREN ZUR HERSTELLUNG VON AMINOETHYL-IMIDAZOLIDINONE ODER DEM THIOCARBONYL DAVON
METHOD FOR PREPARING AMINOETHYL IMIDAZOLIDINONE OR THE THIOCARBONYL THEREOF

(30) Priorité: 11.01.2010 FR 1050127
(43) Date de publication de la demande: 21.11.2012
(73) Titulaire: Arkema France, 92700 Colombes (FR)
(72) Inventeur: BALOCHE, Alain, F-62880 Annay (FR); GAMET, Jean-Paul, F-62690 Savy-Berlette (FR); GILLET, Jean-Philippe, F-69530 Brignais (FR)
(74) Mandataire: Lhoste, Catherine
(86) Numéro de dépôt international: PCT/FR2011/050029
(87) Numéro de publication internationale: WO 2011/083281

(56) Documents cités:
- EP-A1- 0 810 996
- EP-A1- 1 848 776
- US-A- 2 613 212
- US-A- 4 491 527
- US-A- 6 013 200

## Description

La présente invention concerne un procédé de préparation de 1-(2-aminoéthyl)imidazolidin-2-one ou de son thiocarbonyle, et ayant une pureté d'au moins 98%.

La 1-(2-aminoéthyl)imidazolidin-2-one (ci-après, UDETA) est un composé, répondant à la formule suivante : 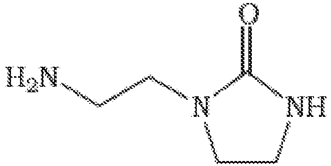 qui a l'avantage de posséder non seulement une fonction amine primaire réactive permettant d'envisager sa condensation sur de nombreux dérivés carbonylés, mais également des doublets d'électrons libres susceptibles de développer des liaisons hydrogènes entre les molécules sur lesquelles elle est greffée ou entre ces molécules et d'autres molécules porteuses de groupes associatifs. L'UDETA est ainsi employée, notamment, pour la synthèse de matériaux supramoléculaires (FR2924715). La Demanderesse a également proposé d'utiliser l'UDETA comme agent salifiant, pour la fabrication d'un additif à base de copolymère styrène / anhydride maléique destiné au couchage ou au collage du papier (FR2925504). L'UDETA intervient également dans la fabrication de composés pharmaceutiques (comme décrit notamment dans WO 2009/142569, US 2009/270372, WO 2009/114566, WO 2009/138438), de produits phytosanitaires, notamment de pesticides, ou d'additifs pour peintures ou lubrifiants (US-5,746,946).

Le brevet US-2 613 212 est l'un des premiers à décrire la synthèse d'UDETA. Dans ce document, il est indiqué que l'UDETA peut être obtenue en faisant réagir au moins une mole de diéthylène triamine (ou DETA) avec une mole d'urée, et de préférence une quantité équimolaire de ces réactifs, à une température de 100 à 300°C. Dans les exemples de ce document, le rapport molaire de la DETA à l'urée est voisin de 1,2. Le mélange est chauffé jusqu'à 210 ou 250°C. Le produit obtenu est ensuite distillé sous pression réduite à 155-163°C. Une application de ce procédé est illustrée à l'Exemple 1 de US-5,746,946, en utilisant un rapport molaire DETA:urée de 1:1 et en utilisant des températures de réaction et de distillation d'au plus 150°C. Il est indiqué dans ce document que le produit obtenu a une pureté de 95%. Les inventeurs ont toutefois démontré, en reproduisant cet exemple, qu'il conduisait à un produit renfermant environ 63% d'UDETA seulement. La différence avec la valeur indiquée dans le brevet US-5,746,946 tient probablement au fait que, dans celui-ci, la pureté est définie par une mesure d'alcalinité, qui n'est pas suffisante pour exclure la présence d'impuretés contenant des fonctions amine et générant elles-mêmes une certaine alcalinité.

De manière analogue, il a été décrit dans US-4 491 527 (Exemple 2) un procédé de synthèse d'UDETA utilisant un mélange de DETA et d'urée dans un rapport molaire DETA:urée de 2:1, qui est porté jusqu'à 203°C. Le produit obtenu est ensuite distillé sous pression réduite, à une température de 165-175°C.

L'Exemple comparatif 1 de la demande DE 199 57 348 divulgue également un procédé de synthèse d'un produit renfermant "majoritairement" de l'UDETA, à partir d'un mélange en quantité équimolaire de DETA et d'urée porté à 155°C. Un autre procédé de ce type est illustré par l'Exemple C de la demande WO 97/49676. Dans cet exemple, le rapport molaire DETA / urée est d'environ 1 et le mélange est chauffé jusqu'à 210°C avant d'être distillé sous pression réduite à plus de 175°C.

L'UDETA obtenue suivant ces procédés renferme, comme l'UDETA commerciale utilisée dans le brevet US-4 104 220, une quantité non négligeable d'impuretés. Les qualités d'UDETA standard disponibles dans le commerce titrent ainsi en général à environ 85% en UDETA.

Pour certaines applications, notamment pour la synthèse de composés supramoléculaires, il serait toutefois souhaitable de pouvoir disposer d'une qualité d'UDETA présentant une pureté d'au moins 90%, par exemple d'au moins 95%. En particulier, si l'UDETA doit être utilisée comme synthon en chimie pharmaceutique, on recherche même une pureté d'au moins 98%, voire d'au moins 99%. Par ailleurs, il est souhaitable de disposer d'une qualité d'UDETA renfermant moins de 5%, voire moins de 3%, en poids de TETU, moins de 1% en poids de DETA, moins de 5% en poids de DETA-urée-DETA et/ou moins de 1% en poids de solvant organique.

Parmi les impuretés majoritairement présentes dans l'UDETA obtenue selon les procédés de l'art antérieur, on peut citer la DETA résiduelle, ainsi que le TETU ou N,N'-bis[2-(2-oxo-1-imidazolidinyl)éthyl] et le produit de condensation DETA-urée-UDETA. La présence de DETA est notamment problématique dans le domaine de la synthèse de composés supramoléculaires, dans la mesure où elle présente plusieurs groupes réactifs susceptibles de former des liaisons indésirables avec les autres synthons utilisés dans la synthèse de ces composés.

Les moyens utilisés jusqu'à présent pour réduire le taux de ces impuretés comprennent notamment la purification de l'UDETA par extraction dans un solvant. La société KING INDUSTRIES propose ainsi un grade d'UDETA plus pur que les grades standard, mais renfermant de grandes quantités de solvant (de l'ordre de 9%) qui peut être préjudiciable dans certaines applications. Un autre moyen de purification de l'UDETA consiste en une distillation sous vide ou pression réduite, qui est habituellement effectuée à une température supérieure à 150°C, et généralement d'au moins 160°C, comme indiqué précédemment. Toutefois, les inventeurs ont démontré que ces conditions de distillation entraînaient une rétrogradation de l'UDETA en DETA et TETU. Précisément, la technique de distillation sous vide classique se traduit par une augmentation de la teneur en TETU en pied de colonne et par l'accumulation d'un mélange UDETA/DETA en tête. Cette rétrogradation affecte la pureté de l'UDETA, ainsi que le rendement de la réaction.

Forts de cette observation, les inventeurs ont ensuite mis en évidence que la pureté de l'UDETA était également affectée par le rapport molaire DETA:urée, qui influence la teneur en TETU. Précisément, plus ce rapport est faible, plus la quantité de TETU formée est importante. A la suite de multiples expériences, ils ont ainsi démontré que l'ajustement de ces deux paramètres (conditions de distillation et rapport DETA:urée) dans des plages données permettait d'obtenir un produit pouvant atteindre une pureté très élevée en UDETA, suivant un procédé simple et économique à mettre en oeuvre, notamment en termes de consommation d'énergie et du fait de l'absence de recours à une étape de purification dans un solvant. Ils ont également démontré que ce procédé pouvait être transposé à la préparation du thiocarbonyle de l'UDETA.

La présente invention a ainsi pour objet un procédé de préparation d'un composé de formule (I) : 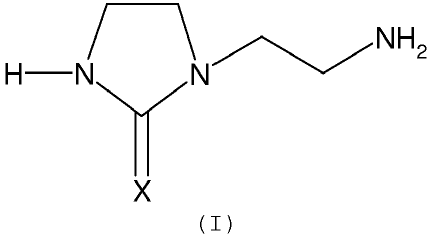 où X est l'oxygène ou le soufre, comprenant les étapes successives consistant à :
1) faire réagir de la diéthylènetriamine (DETA) avec un composé urée choisi parmi l'urée et la thio-urée, dans un rapport molaire DETA / composé urée compris entre 1,2 et 3, à une température d'au plus 300°C,
2) refroidir le milieu réactionnel ;
3) éliminer la DETA résiduelle par distillation sous pression réduite de 1 à 30 mbar à une température, en pied de colonne, comprise entre 100 et 140 °C.

Ce procédé permet d'obtenir un composé ayant une pureté élevée, d'au moins 90%, éventuellement d'au moins 95%, voire d'au moins 98% ou même d'au moins 99%, en ajustant les paramètres de ce procédé. L'UDETA obtenue renferme habituellement moins de 5%, voire moins de 3%, en poids de TETU, moins de 1% en poids de DETA, moins de 5% en poids de DETA-urée-DETA et/ou moins de 1% en poids de solvant organique. Ces valeurs peuvent être mesurées de manière classique pour l'homme du métier, par chromatographie en phase gazeuse (CPG) ou chromatographie liquide haute performance (HPLC).

En outre, le recours à une température de distillation basse présente des avantages en termes d'économie d'énergie, et limite également la dégradation du produit et donc notamment sa coloration.

Dans une forme d'exécution de l'invention, la DETA résiduelle peut en outre être recyclée dans le procédé, compte tenu de sa qualité, et l'ammoniac dégagé par la réaction peut lui-même être récupéré, par exemple pour être envoyé vers un atelier de synthèse d'amines grasses par la voie nitrile, après distillation des eaux ammoniacales. L'économie du procédé se trouve ainsi encore améliorée.

Les étapes du procédé selon l'invention et les réactifs utilisés pour sa mise en oeuvre seront maintenant décrits plus en détail.

A titre de préambule, on notera que l'expression "compris entre" doit être interprétée, dans la présente description, comme incluant les bornes citées.

Dans la première étape du procédé selon l'invention, on fait réagir un composé urée (urée ou thio-urée) avec la DETA, dans un rapport molaire DETA / composé urée compris entre 1,2 et 3,0, de préférence entre 1,3 et 1,7 et plus préférentiellement entre 1,3 et 1,6. A des fins de simplicité, le composé urée peut être introduit sous forme de solution aqueuse, par exemple à 40% dans l'eau. Il est toutefois possible, en variante, d'utiliser l'urée sous forme solide.

Selon une forme d'exécution avantageuse de l'invention, le composé urée est introduit de façon séquencée dans le milieu réactionnel, de préférence par fractions de 10 à 40% en poids par rapport au poids total de composé urée mis en oeuvre. Dans ce cas, la période de temps entre l'introduction de deux fractions successives (avantageusement de masse décroissante) peut être comprise entre 30 min et 1h30, par exemple être d'environ 1h. Cette introduction fractionnée permet de limiter la concentration d'urée libre pouvant conduire à la formation de TETU, de limiter la décomposition thermique de l'urée et d'éliminer lentement l'eau de dilution, sans entraîner trop de DETA. Elle contribue ainsi à augmenter la pureté de l'UDETA obtenue.

Le composé urée peut être mélangé à la DETA avant chauffage du milieu réactionnel. On préfère toutefois que le composé urée soit introduit dans le milieu réactionnel chauffé contenant la DETA.

Ce dernier est chauffé à une température inférieure à 300°C, par exemple comprise entre 100 et 250°C, voire entre 100 et 200°C. La température du milieu réactionnel est avantageusement graduellement augmentée au cours de la réaction, entre la température d'introduction du composé urée et la température finale de la réaction, pour être de préférence portée de 110-140°C à 150-190°C, par exemple par paliers de 10 à 20°C. Le milieu réactionnel peut dans ce cas être maintenu à la température de chaque palier pendant une durée allant de 30 min à 6h. Il est ainsi possible de maîtriser la cinétique de la réaction principale et des réactions parasites et, par suite, de limiter la formation de TETU et de convertir un maximum de la DETA-UREE-DETA, formée comme sous-produit de la réaction, en UDETA.

Le milieu réactionnel est ensuite refroidi, pendant une durée allant par exemple de 30 minutes à 3 heures, puis il est soumis à une étape de distillation à une température, en pied de colonne, comprise entre 100 et 140°C, de préférence entre 120 et 130°C. La distillation est réalisée sous pression réduite, c'est-à-dire sous une pression de 30 à 1 mbar, de préférence de 10 à 1,5 mbar. Elle est par exemple conduite sur une durée maximale de 5 heures.

Il s'agit avantageusement d'une distillation moléculaire.

La distillation moléculaire est une évaporation continue sous vide poussé d'un film mince de produit en mouvement entre une surface chaude (l'évaporateur), dont la surface peut aller par exemple de 0,01 à 50 m², et une surface froide (le condenseur).

La distillation moléculaire peut être mise en oeuvre dans un distillateur à film raclé. Les distillateurs à film raclé comprennent une chambre de distillation dotée d'un racleur tournant, tel qu'un racleur à rouleaux en PTFE/silice, qui permet (notamment sous l'effet de la force centrifuge) l'étalement en continu sur l'évaporateur du produit à distiller. Ces distillateurs opèrent généralement sous une pression de 1 à 100 mbar. Les vapeurs de produit sont évacuées par un orifice situé en partie haute du distillateur avant d'être condensées ou envoyées dans une colonne de rectification. Dans un distillateur à court trajet, les vapeurs sont condensées par le condenseur placé au centre de la chambre de distillation et le vide créé peut aller jusqu'à 0,001 mbar.

Dans tous les cas, la récupération du résidu et du distillat se fait par écoulement gravitationnel.

Des distillateurs moléculaires sont notamment disponibles dans le commerce auprès des sociétés VTA Gmbh, UIC GmbH, FT TECHNOLOGIES et POPE SCIENTIFIC, INC.

On préfère selon l'invention utiliser un procédé de distillation à court trajet et film raclé. Ce procédé permet d'obtenir l'UDETA avec une pureté supérieure à 95%.

Pour augmenter encore la pureté de l'UDETA, le procédé de distillation moléculaire peut avantageusement comprendre les deux étapes successives suivantes :
1- une première distillation à une température de 110°C à 120°C, sous une pression de 1 à 2 mbar, par exemple de 1,5 à 2 mbar,
2- une seconde distillation à une température comprise entre plus de 120°C et 135°C, sous une pression de 0,005 à 0,02 mbar, par exemple de 0,01 à 0,015 mbar.

Cette variante permet d'obtenir un produit particulièrement pur, de pureté supérieure à 99%, avec un rendement (taux de récupération de l'UDETA ou de son thiocarbonyle) de 85 à 90% sans recyclage de la fraction de pied.

Après cette étape de distillation, le procédé selon l'invention comprend généralement une étape de réajustement de la pression à pression atmosphérique et de refroidissement, avant de récupérer le composé produit.

Le procédé selon l'invention permet ainsi d'obtenir un composé ayant une pureté élevée, éventuellement d'au moins 98% ou même d'au moins 99%, en ajustant les paramètres de ce procédé et notamment le rapport molaire DETA / composé urée et les conditions de distillation, comme il ressort des exemples ci-après. A la connaissance des inventeurs, l'UDETA et son dérivé thiocarbonyle n'ont encore jamais été produits avec cette pureté à l'échelle industrielle.

La présente invention permet d'obtenir un composé caractérisé en ce qu'il contient au moins 98% en poids, de préférence au moins 99% en poids de composé de formule (I).

Le produit obtenu selon l'invention peut être utilisé comme synthon ou réactif de synthèse dans de nombreuses applications et notamment pour la fabrication de matériaux supramoléculaires, de composés pharmaceutiques, de produits phytosanitaires, ou d'additifs pour peintures, papier ou lubrifiants.

L'invention sera mieux comprise à la lumière des exemples suivants, donnés à des fins d'illustration seulement et qui n'ont pas pour but de limiter la portée de l'invention, qui est définie par les revendications annexées.

### EXEMPLES

### Exemple 1 : Préparation d'UDETA à partir de DETA et d'urée (R = 1,5)

Dans un réacteur agité de 1 m³, pourvu d'une double enveloppe, d'un dispositif d'introduction de liquide, d'un système d'inertage à l'azote, d'une recette et d'une colonne d'abattage pour récupérer l'ammoniac gazeux et les eaux ammoniacales, on a introduit 693 kg (6728 M) de DETA. On a porté à 130°C le milieu réactionnel tout en effectuant un barbotage d'azote de façon à désoxygéner la DETA. Lorsque la température a été atteinte, on a commencé à introduire une solution aqueuse d'urée à 40%, à raison de 671,4 kg (4476 M). Le rapport molaire DETA:urée était donc de 1,5:1.

L'introduction de l'urée a été réalisée de façon fractionnée sur 4h.

Le dégagement d'ammoniac, apparu dès le début de la coulée, a été abattu sur la colonne d'abattage et les eaux ammoniacales ont été collectées dans la recette. Le mélange réactionnel a ensuite été maintenu pendant 1h30 à 130°C pour achever l'élimination de l'eau.

Le milieu réactionnel a ensuite été chauffé graduellement en le portant : à 140°C pendant 45 min, puis à 150°C pendant 45 min, puis à 160°C pendant 5h. Il a été refroidi à 125°C en fin de réaction, puis la DETA en excès a été éliminée sous pression réduite de 10 à 1,5 mbar et sous léger barbotage d'azote, pendant au maximum 5h. Le produit obtenu après remise à pression atmosphérique de l'équipement, refroidissement vers 60°C et retrait du réacteur titrait à 95-96% en UDETA et contenait 1-2,5% de TETU.

### Exemple 2 : Préparation d'UDETA à partir de DETA et d'urée (R = 1,25)

Le même procédé que celui décrit à l'Exemple 1 a été mis en oeuvre, excepté que le rapport molaire DETA / urée était de 1,25:1. Le produit final titrait à 93% en UDETA et contenait 0,5% de DETA résiduelle et 2,7% de TETU.

### Exemple 3 : Préparation d'UDETA à partir de DETA et d'urée (R = 2)

Le même procédé que celui décrit à l'Exemple 1 a été mis en oeuvre, excepté que le rapport molaire DETA / urée était de 2:1 et que le produit obtenu a été soumis à un strippage sous pression réduite à 120°C au lieu de 125°C. Le produit final titrait à 95% en UDETA et renfermait 0,2% de DETA ; 0,4% de TETU ; et 4,4% d'autres impuretés.

### Exemple 4 : Préparation de thiocarbonyle d'UDETA à partir de DETA et de thiourée (R = 1,5)

Dans un réacteur de 500 cm³, muni d'une agitation mécanique, d'un chauffage, d'un réfrigérant de type Dean Starck, d'une ampoule spécifique à l'introduction de solide, d'un système d'inertage à l'azote et d'une série de flacons laveurs pour piéger l'ammoniac, on a chargé 154,5 g (1,5 M) de DETA. Celle-ci a été portée à 130°C sous balayage d'azote. Puis on a introduit de façon fractionnée sur 1h30 76 g (1 M) de thiourée solide, de sorte que le rapport molaire DETA:thiourée était de 1,5:1.

L'ammoniac se dégageait au fur et à mesure. En fin de coulée, on a porté la température du milieu réactionnel de 130 à 160°C par paliers sur 6h. Après avoir déconnecté les flacons laveurs et installé une pompe à vide, on a procédé à l'élimination de l'excès de DETA en la distillant à 125°C sous 3 mbar.

On a obtenu 143 g d'un brut réactionnel à l'aspect cireux, ayant une pureté en 1-(2-aminoéthyl)-2-imidazolidinethione de 90%.

### Exemple 5 : Distillation moléculaire de l'UDETA

De l'UDETA brute, titrant à 92% et contenant 1% de DETA, a été alimentée à un débit de 681 g/h sur un équipement de distillation à court trajet et film raclé (type VKL 70 de 4 dm² de la société VTA), stabilisé à une pression de 1,9 mbar et à une température de double enveloppe de 120°C. Après environ 2h de distillation, on a obtenu un résidu de 1379 g titrant à 93% en UDETA et renfermant 0,2% de DETA résiduelle, et un distillat de 27,5 g contenant 60% de DETA.

Le résidu a été réintroduit à un débit de 568 g/h dans l'appareil de distillation, après stabilisation de la double enveloppe à 125°C et de la pression à 0,012 mbar. Une seconde distillation a alors été effectuée pendant 38 min dans ces conditions, pour obtenir, d'une part, un distillat incolore de 314 g titrant à 99,8% en UDETA et, d'autre part, un résidu de 45 g titrant à 39% en UDETA et contenant toutes les impuretés lourdes.

En raison de sa très grande pureté, ce distillat peut notamment être utilisé dans la synthèse de produits pharmaceutiques.

### Exemple comparatif 1 : Préparation d'UDETA à partir de DETA et d'urée (R = 1,05)

Dans un réacteur agité de 601, pourvu d'une double enveloppe, d'un dispositif d'introduction de liquide, d'un système d'inertage à l'azote, d'une recette et d'une colonne d'abattage pour récupérer l'ammoniac, on a introduit 42 kg (407,7 M) de DETA. On a porté à 120°C le milieu réactionnel tout en effectuant un barbotage d'azote (0,2 l/s de façon à désoxygéner la DETA. Lorsque la température a été atteinte, on a commencé à introduire une solution aqueuse d'urée à 40%, à raison de 58,4 kg (389 M). Le rapport molaire DETA:urée était donc de 1,05:1.

L'introduction de l'urée a été réalisée de façon fractionnée sur 4h.

Le dégagement d'ammoniac, apparu dès le début de la coulée, a été abattu sur la colonne d'abattage. Le mélange réactionnel a ensuite été maintenu pendant 2h à 120°C pour achever l'élimination de l'eau.

Le milieu réactionnel a ensuite été chauffé graduellement en le portant : à 140°C pendant 2h, puis à 160°C pendant 2h, puis à 175°C pendant 2h puis à 185°C pendant 2h. Il a été refroidi à 125°C en fin de réaction, puis la DETA en excès a été éliminée sous pression réduite. Le produit obtenu après refroidissement vers 60°C et retrait du réacteur titrait à 80-83% en UDETA et contenait 12-13% de TETU. Ce produit présentait donc une pureté très inférieure à 90%.

### Exemple comparatif 2 : Préparation d'UDETA à partir de DETA et d'urée (R = 1, 1)

Le même procédé que celui décrit à l'Exemple 1 a été mis en oeuvre, excepté que le rapport molaire DETA / urée était de 1,1:1 et que le produit obtenu n'a pas été soumis à un strippage sous pression réduite. Le produit final titrait à 81% en UDETA et renfermait 7% de DETA et 9,3% de TETU.

### Exemple comparatif 3 : Distillation classique de l'UDETA

On a soumis le produit issu de l'Exemple comparatif 2 à un procédé de distillation classique.

Le titre théorique en UDETA après élimination de la DETA résiduelle était de 87%. Après distillation à 170°C, sous une pression de 74 mbar, pendant 12 h, on a toutefois obtenu un titre final en UDETA de 64,5% seulement pour ce produit, avec une augmentation de la teneur en TETU jusqu'à 32,6%.

Cet exemple démontre qu'une distillation sous vide dans des conditions classiques, à plus de 150°C, ne permet pas d'obtenir de l'UDETA ayant une pureté d'au moins 90%, et génère au contraire davantage d'impuretés.

### Exemple comparatif 4 : Distillation de l'UDETA à plus de 150°C

On a préparé de l'UDETA suivant un procédé identique à celui de l'Exemple 1, excepté que la distillation sous pression réduite a été effectuée à 180°C. Le produit obtenu présentait la distribution massique suivante, telle que mesurée par RMN :
UDETA : 77,6%
DETA : 1,7%
TETU : 18%
Autres impuretés : 2,7%.

Cet exemple montre qu'un rapport molaire DETA / urée adéquat (compris entre 1,2 et 3,0) n'est pas suffisant pour obtenir un produit ayant la pureté recherchée, et qu'il est en outre nécessaire de limiter la température de distillation à au plus 150°C.

## Revendications

1. Procédé de préparation d'un composé de formule (I) : 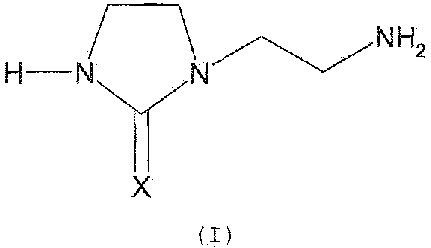
où X est l'oxygène ou le soufre,
comprenant les étapes successives consistant à :
1) faire réagir de la diéthylènetriamine (DETA) avec un composé urée choisi parmi l'urée et la thio-urée, dans un rapport molaire DETA / composé urée compris entre 1,2 et 3, à une température d'au plus 300°C,
2) refroidir le milieu réactionnel ;
3) éliminer la DETA résiduelle par distillation sous pression réduite de 1 à 30 mbar à une température, en pied de colonne, comprise entre 100 et 140°C.

2. Procédé selon la revendication 1, **caractérisé en ce que** le rapport molaire DETA / composé urée est compris entre 1,3 et 1,7 et de préférence entre 1,3 et 1,6.

3. Procédé selon l'une des revendications 1 et 2, **caractérisé en ce que** la distillation est effectuée à une température, en pied de colonne, comprise entre 120 et 130°C.

4. Procédé selon l'une quelconque des revendications 1 à 3, **caractérisé en ce que** la distillation est effectuée sous une pression de 1,5 à 10 mbar.

5. Procédé selon l'une quelconque des revendications 1 à 4, **caractérisé en ce que** la distillation est une distillation moléculaire, de préférence une distillation à court trajet et film raclé.

6. Procédé selon la revendication 5, **caractérisé en ce que** le procédé de distillation moléculaire comprend les deux étapes successives suivantes :
1- une première distillation à une température de 110 à 120°C, sous une pression de 1 à 2 mbar,
2- une seconde distillation à une température comprise entre plus de 120 et 135°C, sous une pression de 0,01 à 0,02 mbar.

7. Procédé selon l'une quelconque des revendications 1 à 6, **caractérisé en ce que** le composé urée est introduit de façon séquencée dans le milieu réactionnel, de préférence par fractions de 10 à 40% en poids par rapport au poids total de composé urée mis en oeuvre.

8. Procédé selon l'une quelconque des revendications 1 à 7, **caractérisé en ce que** le composé urée est introduit dans le milieu réactionnel chauffé contenant la DETA.

9. Procédé selon l'une quelconque des revendications 1 à 8, **caractérisé en ce que** la réaction est effectuée à une température comprise entre 100 et 250°C, de préférence entre 100 et 200°C.

10. Procédé selon la revendication 9, **caractérisé en ce que** le milieu réactionnel est porté, au cours de la réaction, de 110-140°C à 150-190°C.

11. Procédé selon l'une quelconque des revendications 1 à 10, **caractérisé en ce que** la DETA éliminée est recyclée dans le procédé.

## Patentansprüche

1. Verfahren zur Herstellung einer Verbindung der Formel (I): 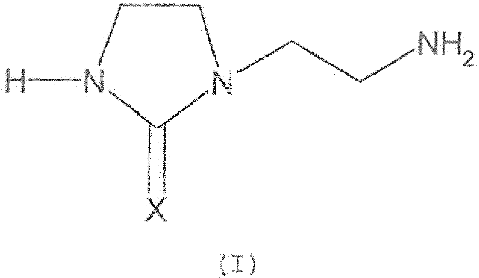
wobei X für Sauerstoff oder Schwefel steht,
mit den aufeinanderfolgenden Schritten, die darin bestehen, dass man:
1) Diethylentriamin (DETA) mit einer aus Harnstoff und Thioharnstoff ausgewählten Harnstoffverbindung in einem Molverhältnis von DETA zu Harnstoffverbindung zwischen 1,2 und 3 bei einer Temperatur von höchstens 300°C umsetzt,
2) das Reaktionsmedium abkühlt;
3) das restliche DETA durch Destillation unter vermindertem Druck von 1 bis 30 mbar bei einer Säulensumpftemperatur zwischen 100 und 140°C entfernt.

2. Verfahren nach Anspruch 1, **dadurch gekennzeichnet, dass** das Molverhältnis von DETA zu Harnstoffverbindung zwischen 1,3 und 1,7 und vorzugsweise zwischen 1,3 und 1,6 liegt.

3. Verfahren nach einem der Ansprüche 1 und 2, **dadurch gekennzeichnet, dass** die Destillation bei einer Säulensumpftemperatur zwischen 120 und 130°C durchgeführt wird.

4. Verfahren nach einem der Ansprüche 1 bis 3, **dadurch gekennzeichnet, dass** die Destillation bei einem Druck von 1,5 bis 10 mbar durchgeführt wird.

5. Verfahren nach einem der Ansprüche 1 bis 4, **dadurch gekennzeichnet, dass** es sich bei der Destillation um eine Molekulardestillation, vorzugsweise eine Wischfilm-Kurzweg-Destillation, handelt.

6. Verfahren nach Anspruch 5, **dadurch gekennzeichnet, dass** das Molekulardestillationsverfahren die folgenden zwei aufeinanderfolgenden Schritte umfasst:
1- eine erste Destillation bei einer Temperatur von 110 bis 120°C unter einem Druck von 1 bis 2 mbar,
2- eine zweite Destillation bei einer Temperatur zwischen mehr als 120 und 135°C unter einem Druck von 0,01 bis 0,02 mbar.

7. Verfahren nach einem der Ansprüche 1 bis 6, **dadurch gekennzeichnet, dass** die Harnstoffverbindung sequenziell in das Reaktionsmedium eingebracht wird, vorzugsweise in Fraktionen von 10 bis 40 Gew.%, bezogen auf das Gesamtgewicht von eingesetzter Harnstoffverbindung.

8. Verfahren nach einem der Ansprüche 1 bis 7, **dadurch gekennzeichnet, dass** die Harnstoffverbindung in das erhitzte Reaktionsmedium, das das DETA enthält, eingebracht wird.

9. Verfahren nach einem der Ansprüche 1 bis 8, **dadurch gekennzeichnet, dass** die Umsetzung bei einer Temperatur zwischen 100 und 250°C, vorzugsweise zwischen 100 und 200°C, durchgeführt wird.

10. Verfahren nach Anspruch 9, **dadurch gekennzeichnet, dass** das Reaktionsmedium im Lauf der Umsetzung von 110-140°C auf 150-190°C gebracht wird.

11. Verfahren nach einem der Ansprüche 1 bis 10, **dadurch gekennzeichnet, dass** das entfernte DETA in das Verfahren zurückgeführt wird.

## Claims

1. A process for preparing a compound of formula (I): 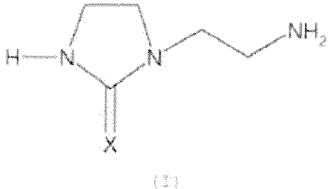
where X is oxygen or sulfur,
comprising the successive steps consisting in:
1) reacting diethylenetriamine (DETA) with a urea compound chosen from urea and thiourea, in a DETA/urea compound molar ratio of between 1.2 and 3, at a temperature of at most 300°C;
2) cooling the reaction medium;
3) removing the residual DETA by distillation under reduced pressure from 1 to 30 mbar at a temperature, at the bottom of the column, of between 100 and 140°C.

2. The process as claimed in claim 1, wherein the DETA/urea compound molar ratio is between 1.3 and 1.7 and preferably between 1.3 and 1.6.

3. The process as claimed in either of claims 1 and 2, wherein the distillation is carried out at a temperature, at the bottom of the column, of between 120 and 130°C.

4. The process as claimed in any one of claims 1 to 3, wherein the distillation is carried out under a pressure of 1.5 to 10 mbar.

5. The process as claimed in any one of claims 1 to 4, wherein the distillation is a molecular distillation, preferably a short-path/wiped-film distillation.

6. The process as claimed in claim 5, wherein the molecular distillation process comprises the following two successive steps:
1- a first distillation at a temperature of from 110°C to 120°C, under a pressure of from 1 to 2 mbar,
2- a second distillation at a temperature between more than 120°C and 135°C, under a pressure of from 0.01 to 0.02 mbar.

7. The process as claimed in any one of claims 1 to 6, wherein the urea compound is introduced sequentially into the reaction medium, preferably in fractions of from 10 to 40% by weight relative to the total weight of urea compound processed.

8. The process as claimed in any one of claims 1 to 7, wherein the urea compound is introduced into the heated reaction medium containing the DETA.

9. The process as claimed in any one of claims 1 to 8, wherein the reaction is carried out at a temperature of between 100 and 250°C, preferably between 100 and 200°C.

10. The process as claimed in claim 9, wherein the reaction medium is brought, during the reaction, from 110-140°C to 150-190°C.

11. The process as claimed in any one of claims 1 to 10, wherein the DETA removed is recycled into the process.
